# EUROPÄISCHE PATENTSCHRIFT

(11) **EP 1 443 893 B1**
(45) Veröffentlichungstag und Bekanntmachung des Hinweises auf die Patenterteilung: **19.09.2012**
(21) Anmeldenummer: 02802994.0
(22) Anmeldetag: 08.11.2002
(51) Int. Cl.: A61Q 15/00, A61K 8/28, A61K 8/39, A61K 8/33, A61K 8/37

(54) **KOSMETISCHE ANTITRANSPIRANT-ROLL-ON-FORMULIERUNGEN AUF W/O-EMULSIONSBASIS**
COSMETIC ANTIPERSPIRANT ROLL-ON FORMULATIONS BASED ON A W/O EMULSION
FORMULATIONS COSMETIQUES ANTITRANSPIRANTES POUR SYSTEMES A BILLE, A BASE D'EMULSION EAU DANS L'HUILE

(30) Priorität: 17.11.2001 DE 10156666
(43) Veröffentlichungstag der Anmeldung: 11.08.2004
(73) Patentinhaber: Cognis IP Management GmbH, 40589 Düsseldorf (DE)
(72) Erfinder: BRÜNING, Stefan, Philadelphia,PA 19118 (US); ANSMANN, Achim, 40699 Erkrath (DE); GONDEK, Helga, D-40589 Düsseldorf (DE)
(86) Internationale Anmeldenummer: PCT/EP2002/012485
(87) Internationale Veröffentlichungsnummer: WO 2003/041674

(56) Entgegenhaltungen:
- WO-A-00/10510
- WO-A-98/55086
- DE-A- 4 420 516
- US-A- 4 203 877
- US-A- 5 534 246
- US-B1- 6 197 283
- US-B1- 6 231 841
- US-B1- 6 241 976
- US-B1- 6 248 312
- PATENT ABSTRACTS OF JAPAN vol. 016, no. 485 (C-0993), 8. Oktober 1992 (1992-10-08) & JP 04 178316 A (KAO CORP), 25. Juni 1992 (1992-06-25)
- PATENT ABSTRACTS OF JAPAN vol. 009, no. 052 (C-269), 6. März 1985 (1985-03-06) & JP 59 193847 A (NISSHIN SEIYU KK), 2. November 1984 (1984-11-02)

## Beschreibung

### Gebiet der Erfindung

Gegenstand der vorliegenden Erfindung sind Antitranspirant-Roll-on-Formulierungen auf Basis von Wasser in Öl-Emulsionen.

### Stand der Technik

Auf dem Markt sind zahlreiche Applikationsformen für Antitranspirantien üblich, beispielsweise Spray, Cremes, Stifte sowie Roll-on-Formulierungen auf Basis von O/W-Emulsionen.

Antitranspirant-Roll-on-Formulierungen auf Basis von O/W-Emulsionen zeigen den Nachteil, dass sie häufig stark weißeln, d. h. zur Mikroschaumbildung neigen und Stabilitätsprobleme aufweisen.

Aus der EP 998 909 A1 sind W/O-Antitranspirant-Formulierungen auf Basis bestimmter Alaune bekannt. Diese zeigen jedoch nur eine begrenzte Deo-Leistung. Aus der DE 24 08 663 ist eine schweißhemmende, wasserfeste Creme bekannt. Aus der US 5,534,246 sind Wasser-in-Öl-Antitranspirant-Roll-ons bekannt, die als Emulgatoren polyoxyethylierte C₆-C₂₂-Fettalkohole, ethoxylierte Alkylphenole, sowie Polyethylenglycolether von Methylglucose oder Sorbitol enthalten. Diese Formulierungen sind hinsichtlich der Langzeitstabilität, insbesondere auch unter Temperaturbelastung verbesserungswürdig.

Aufgabe der vorliegenden Erfindung war es daher, einerseits das Weißeln in Antitranspirant-Roll-on-Formulierungen zu reduzieren, d.h. dünnflüssige Formulierungen zur Verfügung zu stellen, die eine geringere Neigung zur Mikroschaumbildung haben, und trotz der Gegenwart antitranspiranter Wirkstoffe sehr gut hautverträglich sind. Die Formulierungen sollten darüber hinaus eine reduzierte Klebrigkeit aufweisen und ein trockenes Hautgefühl vermitteln, schnell absorbiert werden, und trotz hoher Salzbelastung auch bei Temperaturbelastung eine sehr hohe Stabilität aufweisen.

### Beschreibung der Erfindung

Gegenstand der vorliegenden Erfindung sind W/O-Emulsionen mit einer Viskosität von 500 - 10000 mPa·s bei 20°C (Brookfield RVT, Spindel TA, 20 rpm, mit Helipath) enthaltend (a) wenigstens einen Antitranspirant-Wirkstoff, (b) wenigstens einen W/O-Emulgator ausgewählt aus der Gruppe der Polyolpoly-12-hydroxystearate, (c) eine Ölphase und (d) Wasser.

Überraschenderweise wurde gefunden, dass Antitranspirant-Roll-on-Formulierungen auf Basis von W/O-Emulsionen im Vergleich zu O/W-Emulsionen eine deutlich reduzierte Mikroschaumbildung zeigen. Durch Einsatz ganz spezieller W/O-Emulgatoren, der Polyolpoly-12hydroxystearate, weisen die erfindungsgemäßen Zusammensetzungen auch bei vergleichsweise hoher Konzentration der antitranspiranten Wirkstoffe, also bei hoher Salzbelastung, eine ausgezeichnete Stabilität auf und sind sehr gut hautverträglich. Die erfindungsgemäßen W/O-Emulsionen werden in Roll-on Applikatoren eingesetzt. In einer bevorzugten Ausführungsform weisen sie eine Viskosität von 500 - 5000 mPa·s bei 20 °C auf (Brookfield RVT, Spindel TA, 20 rpm, mit Helipath).

### Antitranspirant-Wirkstoffe

Erfindungsgemäß eignen sich als Antitranspirant-Wirkstoffe wasserlösliche, adstringierende metallische Salze, insbesondere anorganische und organische Salze des Aluminiums, Zirkoniums und Zinks bzw. Mischungen dieser Salze, beispielsweise Aluminiumchlorhydrate und/oder Aluminium-ZirkoniumChlorohydrate. Diese wirken wahrscheinlich über den partiellen Verschluß der Schweißdrüsen durch Eiweiß- undloder Polysaccharidfällung. Neben den Chlorhydraten können auch Aluminiumhydroxylactate sowie saure Aluminium/Zirkoniumsalze eingesetzt werden. Unter der Marke Locron^{®} der Clariant GmbH, befindet beispielsweise sich ein Aluminiumchlorhydrat im Handel, das der Formel [Al₂(OH)₅Cl]·2,5 H₂O entspricht, und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal^{®} 36G vermarktet werden. Die erfindungsgemäßen Zubereitungen enthalten als Antitranspirant-Wirkstoff vorzugsweise wenigstens ein Aluminiumsalz, und insbesondere ein Aluminium-Zirkonium-Tetrachlorohydrex-Glycin-Komplex. Das Aluminiumsalz ist üblicherweise in einer Konzentration von 1 - 30 Gew.-%, vorzugsweise 5 - 25 Gew.-% und insbesondere 5 - 20 Gew.-% enthalten.

### Polyolpoly-12-hydroxystearate

Bei den Polyolpoly-12-hydroxystearaten, welche die Komponente (b) bilden, handelt es sich um bekannte Stoffe. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methylolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit;
(f) Zucker mit 5 bis 12 Kohlenstoffatomen, wie beispielsweise Glucose oder Saccharose;
(g) Aminozucker, wie beispielsweise Glucamin.

Unter den erfindungsgemäß einzusetzenden Emulgatoren kommt Umsetzungsprodukten auf Basis von Polyglycerin und Polyethylenglykol wegen ihrer ausgezeichneten anwendungstechnischen Eigenschaften eine besondere Bedeutung zu. Als besonders vorteilhaft haben sich Umsetzungsprodukte von Poly-12-hydroxystearinsäure mit Polyglycerinen folgender Homologenverteilung erwiesen (bevorzugte Mengen sind in Klammem angegeben):

| | |
|---|---|
| Glycerine | : 5 bis 35 (15 bis 30) Gew.-% |
| Diglycerine | : 15 bis 40 (20 bis 32) Gew.-% |
| Triglycerine | :10 bis 35 (15 bis 25) Gew.-% |
| Tetraglycerine | : 5 bis 20 (8 bis 15) Gew.-% |
| Pentaglycerine | : 2 bis 10 (3 bis 8) Gew.-% |
| Oligoglycerine | : ad 100 Gew.-% |

Erfindungsgemäß besonders vorteilhaft ist der Einsatz eines Poly(12-hydroxystearinsäure)polyglycerinesters, der unter der Bezeichnung D/ehymuls^{®} PGPH (INCI: Polyglyceryl-2 Dipolyhydroxystearate) von der Cognis Deutschland GmbH & Co. KG vertrieben wird. Ebenso vorteilhaft ist der Einsatz eines PEG-30 Dipolyhydroxystearats. In einer besonders bevorzugten Ausführungsform enthält die erfindungsgemäße Zusammensetzung eine Kombination aus einem PEG-30 Dipolyhydroxystearat und einem Poly(12-hydroxystearinsäure)polyglycerinester, also beispielsweise Arlacel^{®} P 135 und Dehymuls^{®} PGPH. Ein weiterer Gegenstand der Erfindung ist die Verwendung von Polyol-12-hydroxystearaten zur Verminderung der Mikroschaumbildung in Antitranspirant-Formulierungen.

Die Polyolpoly-12-hydroxystearate sind in den erfindungsgemäßen Emulsionen üblicherweise in einer Menge 1-10 Gew.-%, vorzugsweise 2-8 Gew.-% und besonders bevorzugt 3-7 Gew.-% enthalten.

### Ölkörper

Die erfindungsgemäßen W/O-Emulsionen enthalten eine Ölphase, die sich aus einem einzelnen oder einem Gemisch von Ölkörpern zusammensetzen kann. Unter Ölkörpem sind erfindungsgemäß bei 20 °C flüssige, mit Wasser bei 25 °C nicht mischbare Stoffe oder Gemische von Stoffen zu verstehen. Die Ölkörper sind in den erfindungsgemäßen Emulsionen üblicherweise in Mengen von 5-30 Gew.-%, vorzugsweise 5 - 25 Gew.-% und besonders bevorzugt 10-20 Gew.-% enthalten.

Als Ölkörper kommen beispielsweise Guerbetalkohole auf Basis von Fettalkoholen mit 6 bis 18, vorzugsweise 8 bis 10 Kohlenstoffatomen (z. B. Eutanol^{®} G), Ester von linearen C₆-C₂₂-Fettsäuren mit linearen oder verzweigten C₆-C₂₂-Feftalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen - insbesondere Diethylhexylmalat -, Ester von linearen undloder verzweigten Fettsäuren mit mehrwertigen Alkoholen (wie z.B. Propylenglycol, Dimerdiol oder Trimertriol) undloder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen (Hydagen^{®} HSP, Sovermol^{®} 750, Sovermol^{®} 1102), Siliconöle (Cyclomethicone, Siliciummethicontypen u.a.) undloder aliphatische bzw. naphthenische Kohlenwasserstoffe, wie z.B. wie Mineralöl, Vaseline, Petrolatum, Squalan, Squalen oder Dialkylcyclohexane in Betracht.

Eine deutliche Verbesserung der sensorischen Eigenschaften, insbesondere eine im Vergleich zu O/W-Roll-on-Emulsionen schnellere Absorption des Antitranspirantsalzes, eine reduzierte Klebrigkeit und ein geringeres Haut-Irritationspotential der erfindungsgemäßen W/O-Emlusionen resultiert, wenn Dialkylether undloder Dialkylcarbonate als Olkörper eingesetzt oder mitverwendet werden. In einer bevorzugten Ausführungsform der Erfindung enthält die Ölphase wenigstens eine Ölkomponente ausgewählt aus der Gruppe der Dialkyl(en)ether, der Dialkyl(en)carbonate oder eines Gemisches dieser Substanzen.

Die **Dialk(en)ylether** können symmetrisch oder unsymmetrisch, verzweigt oder unverzweigt, gesättigt oder ungesättigt sein. Erfindungsgemäß bevorzugt geeignet sind gesättigte C₆-C₃₂-Dialkylether, wie beispielsweise Di-n-octylether, Di-(2-ethylhexyl)-ether, Laurylmethylether oder Octylbutylether, sowie Didodecylether. Die Verbindungen lassen sich aus Fettalkoholen in Gegenwart saurer Katalysatoren nach allgemein bekannten Verfahren des Standes der Technik herstellen, z. B. DE 19511668 A1 und DE 19831705 A1 sowie DE 19943585. Typische Beispiele für derartige Ether sind Produkte, die durch Veretherung von Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestern auf Basis von Fetten und Ölen anfallen, gewonnenen werden.

Die **Dialk(en)ylcarbonate** können symmetrisch oder unsymmetrisch, verzweigt oder unverzweigt, gesättigt oder ungesättigt sein. Unter den Dialk(en)ylcarbonaten sind lineare oder verzweigte, gesättigte oder ungesättigte C₆-C₃₂-Dialkyl(en)carbonate erfindungsgemäß bevorzugt, wie z. B. Dihexyl-, Dioctyl-, Di-(2-ethylhexyl)- oder Dioleylcarbonat. Die Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in **Chem.Rev. 96, 951 (1996)**. Typische Beispiele für Dialkyl(en)carbonate sind Umesterungsprodukte von Dimethyl- und/oder Diethylcarbonat mit Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestern auf Basis von Fetten und Ölen anfallen.

Der Zusatz von Siliconverbindungen zu den erfindungsgemäßen Zusammensetzungen vermittelt in der Applikation ein sensorisch leichteren Eindruck. Eine weitere bevorzugte Ausführungsform der Erfindung ist daher dadurch gekennzeichnet, daß die Ölphase wenigstens eine bei 20° C flüssige Silikonverbindung enthält.

Geeignete Siliconverbindungen sind beispielsweise Dimethylpolysiloxane und cyclische Silicone sowie deren ggf. substituierte Analoga. Hierzu zählen beispielsweise DM-Fluid^{®} 0.6cs, DM-Fluid^{®} 1.0cs, DM-Fluid^{®} 1.5cs, DM-Fluid^{®} 2.0cs, DM-Fluid^{®} 10cs, DM-Fluid^{®} 100cs, DM-Fluid^{®} 500cs, die von Shin Etsu Silicones of America, Inc. vetrieben werden.

Beispielsweise können Cyclomethicone und Dimethicone in Mengen von 0,5 - 20 Gew.-%, vorzugsweise 1 - 15 Gew.-% und insbesondere 1 - 13 Gew.-% bezogen auf die Gesamtzusammensetzung eingesetzt werden. Der Zusatz von Dimethicone trägt zur weiteren Reduktion der Mikroschaumbildung bei.

Eine bevorzugte Ausführungsform der W/O-Emulsion enthält (a) 1 - 30 Gew.-% wenigstens eines Aluminiumsalzes, (b) 1 - 10 Gew.-% wenigstens eines W/O-Emulgators ausgewählt aus der Gruppe Ester der 12-Hydroxystearinsäure mit Polyethylenglykolen oder Polyglycerinen oder eines Gemisches dieser Ester, (c) 5 - 30 Gew.-% einer Ölphase und (d) 40 - 90 Gew.-% Wasser. Vorzugsweise enthält die W/O-Emulsion (a) 5 - 25 Gew.-% Gew.-% wenigstens eines Aluminiumsalzes, (b) 2 - 8 Gew.-% wenigstens eines W/O-Emulgators ausgewählt aus der Gruppe Ester der 12-Hydroxystearinsäure mit Polyethylenglykolen oder Polyglycerinen oder eines Gemisches dieser Ester, (c) 5 - 25 Gew.-% einer Ölphase und (d) 40 - 80 Gew.-% Wasser. Besonders bevorzugt ist eine W/O-Emulsion mit (a) 5 - 20 Gew.-% Gew.-% wenigstens eines Aluminiumsalzes, (b) 3 - 7 Gew.-% Gew.-% wenigstens eines W/O-Emulgators ausgewählt aus der Gruppe Ester der 12-Hydroxystearinsäure mit Polyethylenglykolen oder Polyglycerinen oder eines Gemisches dieser Ester, 10-20 Gew.-% einer Ölphase und (d) 40 - 70 Gew.-% Wasser. Die Ölphase enthält vorzugsweise Dialkylcarbonate, Dialkyether oder ein Gemisch dieser Substanzen, besonders bevorzugt in Kombination mit einer unter Normaldruck bei 20° C flüssigen Silikonverbindung.

### Zusätzliche Deo-Wirkstoffe

Die erfindungsgemäßen W/O-Emulsionen können zusätzliche Deowirkstoffe wie z.B. Esteraseinhibitoren enthalten. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw - phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Zusammensetzungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan^{®} von der Ciba-Geigy, Basel/CH vertrieben wird.

Auch bakterizide bzw. bakteriostatische Wirkstoffe können zusätzlich enthalten sein, um die Deo-Wirkung zu verbessern. Hierzu gehören beispielsweise Chitosan, Phenoxyethanol oder 5-Chlor-2-(2,4-dichlorphenoxy)phenol, das unter der Marke Irgasan® von der Ciba-Geigy, Basel/CH vertrieben wird, sowie antibakteriell wirksame Riechstoffe wie z. B. Thymol, Thymianöl, Eugenol, Nelkenöl, Menthol, Minzöl, Farnesol, und antibakteriell wirksame Glycerinester und -ether wie z. B: Glycerinmonocaprinat, Glycerinmonocaprylat, Glycerinmonolaurat (GML) und Diglycerinmonocaprinat (DMC).

Auch der weiterer Zusatz von Geruchsabsorbem kann eine Steigerung der Deo-Leistung bewirken. Geruchsabsorber haben keine Wirksamkeit gegen Bakterien,- sondern verringern lediglich den Partialdruck der geruchsbildenden Komponenten. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind.

Als schweißabsobierende Substanzen kommen beispielsweise modifizierte Stärke, wie z.B. Dry Flo^{®} Plus (Fa. National Starch), Silikate und Talkum in Frage. Als Riechstoffe, die zur Geruchsüberdeckung eingesetzt werden, eignen sich pflanzliche, tierische und synthetische Riechstoffe.

### Feuchthaltemittel/Hautbefeuchtungsmittel

In einer weiteren bevorzugten Ausführungsform enthält die W/O-Emulsion daher wenigstens ein Feuchthaltemittel. Diese tragen zur Verbesserung der Kältestabilität der erfindungsgemäßen W/O-Emulsionen bei. Feuchthaltemittel sind üblicherweise in einer Menge von 0,1-10Gew.-%, vorzugsweise 0,5-10 Gew.-%, und insbesondere 0,5 - 6 Gew.-% bezogen auf die W/O-Emulsion enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Polyethylenglycole, Glycerin, Diglycerin und Triglycerin.

### Kieselsäuren

Eine weitere Verbesserung der sensorischen Eigenschaften (insbesondere trockenes Hautgefühl) kann durch Einarbeitung von Kieselsäuren erreicht werden, unter denen Verbindungen der allgemeinen Formel SiO₂·nH₂O zu verstehen sind. Erfindungsgemäß bevorzugt sind hochdisperse Kieselsäuren, insbesondere Aerosil^{®}. In einer weiteren bevorzugten Ausführungsform enthält die W/O-Emulsion daher Kieselsäuren, vorzugsweise in Mengen von 0,1-3,0 Gew.-%.

### Alkali/Erdalkalimetallsalze

Eine weitere bevorzugte Ausführungsform der erfindungsgemäßen W/O-Emulsion enthält wenigstens ein anorganisches Salz ausgewählt aus den Alkalimetall- oder Erdalkalimetallchloriden oder -sulfaten oder eines beliebigen Gemisches dieser Salze enthalten ist. Dieses trägt zur Erhöhung der Emulsionsstabiltät sowie der Kältestabiltät der erfindungsgemäßen W/O-Emulsion bei. Vermutlich wird dies dadurch erzielt, daß ein Migrieren des Emulgators von der Grenzschicht in die innere, wässrige Phase durch den Salzgehalt deutlich verringert wird. Erfindungsgemäß einsetzbar sind wasserlösliche Salze, wie beispielsweise Natriumchlorid, Natriumsulfat, Kaliumsulfat, Magnesiumchlorid, Calciumchlorid oder Magnesiumsulfat bzw. beliebige Mischungen dieser Salze.

### Weitere Hilfs- und Zusatzstoffe

Die erfindungsgemäßen W/O-Emulsionen können eine Reihe weiterer Hilfs- und Zusatzstoffe enthalten, wie beispielsweise wie beispielsweise weitere Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antioxidantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe etc.

### Erfindungsgemäße Beispiele 1-7 und Vergleichsbeispiel V1

| **Zusammensetzung (Gew.%)** | **INCI** | **V 1** | **1** | **2** | **3** | **4** | **5** | **6** | **7** |
|---|---|---|---|---|---|---|---|---|---|
| Eumulgin S2 | Steareth-2 | 3,0 | | | | | | | |
| Eumulgin S21 | Steareth-21 | 2,0 | | | | | | | |
| Dehymuls^{®} PGPH | Polyglyceryl-2 Dipolyhydroxystearat | | 2,0 | 2,0 | | 2,0 | 2,0 | 2,0 | 2,0 |
| Arlacel^{®} P 135 | PEG-30 Dipolyhydroxystearat | | 2,0 | 2,2 | 4,4 | 2,2 | 2,2 | 2,2 | 2,2 |
| Cetiol^{®} OE | Dicaprylyl Ether | 1,0 | 4,5 | 4,5 | 4,5 | 4,5 | 4,5 | 4,5 | 4,5 |
| Cetiol^{®} CC⁻ | Dicaprylyl Carbonate | 1,0 | 4,5 | 4,1 | 3,7 | 4,1 | 4,1 | 4,1 | 4,1 |
| Cetiol^{®} SN | Cetearyl Isononanoate⁻ | | 4,8 | | | | | | |
| Dow Corning^{®} 245 | Cyclomethicone | 1,5 | - | 7,0 | 7,0 | 7,0 | 12,0 | 7,0 | 7,0 |
| Eutanol^{®} G16 | Hexyldecanol | | 2,2 | | | | | | |
| Glycerin | | | 5,0 | 5,0 | 5,0 | 5,0 | 3,0 | 5,0 | 5,0 |
| Magnesiumsulfat 7H20 | | 0,8 | 1,0 | 1,0 | 1,0 | 1,0 | 1,0 | 1,0 | 1,0 |
| Aerosil^{®} R 972 | Silica Dimethyl Silylate | | | | | | 0,5 | 0,5 | 1,5 |
| Hydagen^{®} CAT | Triethyl Citrate | | | | | 1,0 | | | |
| Irgasan^{®} DP 300 | Triclosan | | | | | 0,5 | | | |
| Hydagen^{®} DCMF | Chitosan | | | | | 0,05 | | | |
| Rezal^{®} 36 GC | Aluminium Zirconium Tetrachlorohydrex Glycine | | 40,0 | 40,0 | | | | | |
| Locron^{®} L | Aluminum Chlorohydrat | 40,0 | | | 20,0 | | | 20,0 | 20,0 |
| Locron^{®} P | Aluminum Chlorohydrat | | | | | | 20,0 | | |
| Wasser | | ad 100 | Ad 100 | ad 100 | ad 100 | ad 100 | ad 100 | ad 100 | ad 100 |
| **Bewertung** | | | | | | | | | |
| *Mikroschaumbildung* | | - | + | + | + | + | + | + | + |
| *Stabilität* | | - | + | + | + | + | + | + | + |
| *Absorption* | | - | + | + | + | + | + | + | + |
| *Klebrigkeit* | | 0 | + | + | + | + | + | + | + |
| *Trockenheit* | | 0 | 0 | 0 | 0 | 0 | + | + | + |
| *Viskosität: in mPa·s bei 20 C°* | | 1750 | 1500 | 2000 | 2000 | 1500 | 2750 | 2500 | 3000 |

| | | | | | | | | | |
|---|---|---|---|---|---|---|---|---|---|
| Bewertung: - (schlecht); 0 (mittel); + (gut) (Brookfield RVT, Spindel TA, 20 rpm, mit Helipath) | | | | | | | | | |

**Anhand**
1) Aerosil^{®} R972
   INCI: Silica Dimethyl Silylate
   Hersteller: Degussa AG
2) Arlacel^{®} P 135
   INCI: PEG-30 Dipolyhydroxystearate
   Hersteller: Uniqema (ICI Surfacants)
3) Cetiol^{®} CC
   INCI: Dicaprylyl Carbonate
   Hersteller: Cognis Deutschland GmbH
4) Cetiol^{®} OE
   INCI: Dicaprylyl Ether
   Hersteller: Cognis Deutschland GmbH
5) Cetiol^{®} SN
   INCI: Cetearyl Isononanoate
   Hersteller: Cognis Deutschland GmbH
6) Dehymuls^{®} PGPH
   INCI: Polyglyceryl-2 Dipolyhydroxystearate
   Hersteller: Cognis Deutschland GmbH
7) Dow Coming^{®} 245
   INCI: Cyclomethicone
   Hersteller: Dow Corning
8) Eumulgin^{®} S 2
   INCI: Steareth-2
   Hersteller: Cognis Deutschland GmbH
9) Eumulgin^{®} S 21
   INCI: Steareth-21
   Hersteller: Cognis Deutschland GmbH
10) Eutanol^{®} G 16
   INCI: Hexyldecanol
   Hersteller: Cognis Deutschland GmbH
11) Hydagen^{®} DCMF
   INCI: Chitosan
   Hersteller: Cognis Deutschland GmbH
12) Hydagen^{®} CAT
   INCI: Triethyl Citrate
   Hersteller: Henkel KGaA
13) Irgasan^{®} DP 300
   INCI: Triclosan
   Hersteller: Ciba Specialty Chemicals Inc.
14) Locron^{®} L
   INCI: Aluminium Chlorohydrat
   Hersteller: Clariant GmbH
15) Locron^{®} P
   INCI: Aluminium Chlorohydrat
   Hersteller: Clariant GmbH
16) Rezal^{®} 36 GC
   INCI: Aluminium Zirconium Tetrachlorohydrex Glycine
   Hersteller: Reheis Inc.

## Patentansprüche

1. Kosmetsiche W/O-Emulsion mit einer Viskosität von 500 -10000 mPa·s bei 20 °C enthaltend
(a) wenigstens einen Antitranspirant-Wirkstoff
(b) wenigstens einen W/O-Emulgator ausgewählt aus der Gruppe Polyolpoly-12-hydroxystearate
(c) eine Ölphase und
(d) Wasser.

2. W/O-Emulsion gemäß Anspruch 1, **dadurch gekennzeichnet, dass** sie bei 20°C eine Viskosität von 500 - 5000 mPa·s aufweisen.

3. W/O-Emulsion gemäß Anspruch 1 oder 2, **dadurch gekennzeichnet, dass** als Antitranspirant-Wirkstoff (a) wenigstens ein Aluminiumsalz enthalten ist.

4. W/O-Emulsion gemäß wenigstens einem der Ansprüche 1 bis 3, **dadurch gekennzeichnet, dass** sie
(a) 1 - 30 Gew.% wenigstens eines Aluminiumsalzes
(b) 1 - 10 Gew.-% wenigstens eines W/O-Emulgators ausgewählt aus der Gruppe Ester der 12-Hydroxystearinsäure mit Polyethylenglykolen oder Polyglycerinen oder eines Gemisches dieser Ester
(c) 5 - 30 Gew.-% einer Ölphase und
(d) 40 - 90 Gew.-% Wasser
enthält.

5. W/O-Emulsion gemäß wenigstens einem der Ansprüche 1 bis 4, **dadurch gekennzeichnet, dass** die Ölphase (c) wenigstens eine Ölkomponente ausgewählt aus der Gruppe der Dialkyl(en)ether, der Dialkyl(en)carbonate oder eines Gemisches dieser Komponenten enthält.

6. W/O-Emulsion gemäß wenigstens einem der Ansprüche 1 bis 5, **dadurch gekennzeichnet, dass** die Ölphase (c) wenigstens eine Silikonverbindung enthält.

7. W/O-Emulsion gemäß wenigstens einem der Ansprüche 1 bis 6, **dadurch gekennzeichnet, dass** zusätzlich wenigstens ein Feuchthaltemittel, vorzugsweise Glycerin, enthalten ist.

8. W/O-Emulsion gemäß wenigstens einem der Ansprüche 1 bis 7, **dadurch gekennzeichnet, dass** zusätzlich wenigstens eine Kieselsäure enthalten ist.

9. W/O-Emulsion gemäß wenigstens einem der Ansprüche 1 bis 8, **dadurch gekennzeichnet, dass** zusätzlich wenigstens ein anorganisches Salz ausgewählt aus den Alkalimetall- oder Erdalkalimetallchloriden oder -sulfaten oder eines beliebigen Gemisches dieser Salze enthalten ist.

10. Verwendung von Poyolpoly-12-hydroxystearaten zur Verminderung der Mikroschaumbildung in Antitranspirant-Zusammensetzungen.

## Claims

1. A cosmetic w/o emulsion with a viscosity of 500 to 10,000 mPa·s at 20°C containing
(a) at least one antiperspirant component,
(b) at least one w/o emulsifier selected from the group of polyolpoly-12-hydroxystearates,
(c) an oil phase and
(d) water.

2. A w/o emulsion as claimed in claim 1, **characterized in that** they have a viscosity at 20°C of 500 to 5,000 mPa·s.

3. A w/o emulsion as claimed in claim 1 or 2, **characterized in that** at least one aluminium salt is present at the antiperspirant component (a).

4. A w/o emulsion as claimed in at least one of claims 1 to 3, **characterized in that** it contains
(a) 1 to 30% by weight of at least one aluminium salt,
(b) 1 to 10% by weight of at least one w/o emulsifier selected from the group of esters of 12-hydroxystearic acid with polyethylene glycols or polyglycerols or a mixture of these esters,
(c) 5 to 30% by weight of an oil phase and
(d) 40 to 90% by weight of water.

5. A w/o emulsion as claimed in at least one of claims 1 to 4, **characterized in that** the oil phase (c) contains at least one oil component selected from the group of alkyl(ene) ethers, dialkyl(ene) carbonates or a mixture of these components.

6. A w/o emulsion as claimed in at least one of claims 1 to 5, **characterized in that** the oil phase (c) contains at least one silicone compound.

7. A w/o emulsion as claimed in at least one of claims 1 to 6, **characterized in that** at least one humectant, preferably glycerol, is additionally present.

8. A w/o emulsion as claimed in at least one of claims 1 to 7, **characterized in that** at least one silica is additionally present.

9. A w/o emulsion as claimed in at least one of claims 1 to 8, **characterized in that** at least one inorganic salt selected from the alkali metal or alkaline earth metal chlorides or sulfates or a mixture of these salts is additionally present.

10. The use of polyolpoly-12-hydroxystearates for reducing the formation of microfoam in antiperspirant compositions.

## Revendications

1. Émulsion E/H cosmétique ayant une viscosité de 500 - 10 000 mPa.s à 20°C, contenant
(a) au moins une substance active antisudorale
(b) au moins un émulsifiant E/H choisi dans le groupe des polyolpoly-12-hydroxystéarates
(c) une phase huileuse et
(d) de l'eau.

2. Émulsion E/H selon la revendication 1, **caractérisée en ce qu'**elle présente à 20°C une viscosité de 500 - 5 000 mPa.s.

3. Émulsion E/H selon la revendication 1 ou 2, **caractérisée en ce que** comme substance active antisudorale (a) est contenu au moins un sel d'aluminium.

4. Émulsion E/H selon au moins l'une quelconque des revendications 1 à 3, **caractérisée en ce qu'**elle contient
(a) 1 - 30 % en poids d'au moins un sel d'aluminium
(b) 1 - 10 % en poids d'au moins un émulsifiant E/H choisi dans le groupe constitué par les esters de l'acide 12-hydroxystéarique avec des polyéthylèneglycols ou des polyglycérols ou un mélange de ces esters
(c) 5 - 30 % en poids d'une phase huileuse et
(d) 40 - 90 % en poids d'eau.

5. Émulsion E/H selon au moins l'une quelconque des revendications 1 à 4, **caractérisée en ce que** la phase huileuse (c) contient au moins un composant huileux choisi dans le groupe constitué par les dialkyl(ène)éthers, les dialkyl(ène)carbonates ou un mélange de ces composants.

6. Émulsion E/H selon au moins l'une quelconque des revendications 1 à 5, **caractérisée en ce que** la phase huileuse (c) contient au moins un composé silicone.

7. Émulsion E/H selon au moins l'une quelconque des revendications 1 à 6, **caractérisée en ce qu'**en outre au moins un humectant, de préférence du glycérol, est contenu.

8. Émulsion E/H selon au moins l'une quelconque des revendications 1 à 7, **caractérisée en ce qu'**en outre au moins un acide silicique est contenu.

9. Émulsion E/H selon au moins l'une quelconque des revendications 1 à 8, **caractérisée en ce qu'**en outre au moins un sel inorganique choisi parmi les chlorures ou sulfates de métaux alcalins ou de métaux alcalino-terreux ou un mélange quelconque de ces sels est contenu.

10. Utilisation de polyolpoly-12-hydroxystéarates pour réduire la formation de micromousses dans des compositions antisudorales.
